# EUROPEAN PATENT APPLICATION

(11) **EP 4 098 246 A1**
(43) Date of publication of application: **07.12.2022**
(21) Application number: 21176831.2
(22) Date of filing: 31.05.2021
(51) Int. Cl.: A61K 9/00, A61K 9/48, A61K 9/50, A61K 47/14, A61K 47/44

(54) **FORMULATION OF NINTEDANIB**

(71) Applicant: Lotus Pharmaceutical Co., Ltd., 11046 Taipei City (TW)
(72) Inventor: Shih, Stone, 54066 Nantou City (TW); Mali, Rahul Krishnat, 54066 Nantou City (TW); Gupta, Vijender, 54066 Nantou City (TW); Chawla, Manish, 54066 Nantou City (TW)
(74) Representative: Hartvichova, Katerina

(57) **Abstract**

The present invention relates to a formulation of nintedanib or a pharmaceutically acceptable salt thereof which contains a non-ionic surfactant. The formulation has an immediate release profile without the need to use lecithin as a surfactant. The formulation is suitable for filling into capsules.

## Description

### Field of Art

The present invention relates to novel formulations of nintedanib or pharmaceutically acceptable salt thereof, to capsules comprising such formulations, and to their medical uses.

### Background Art

Nintedanib is a pharmaceutically active substance which acts by inhibiting non-receptor tyrosine kinases and receptor tyrosine kinases, including Lck, Lyn, Src, PDGFR, FGFR, VEGFR, FLT3. The chemical name of nintedanib is methyl (3Z)-3-{[(4-{methyl[(4-methylpiperazin-1-yl)acetyl]amino}phenyl)amino](phenyl)methylidene}-2-oxo-2,3-dihydro-1H-indole-6-carboxylate. Nintedanib is commercially available as Ofev and Vargatef. Vargatef is indicated in combination with docetaxel for the treatment of adult patients with locally advanced, metastatic or locally recurrent non-small cell lung cancer (NSCLC) of adenocarcinoma tumour histology after first-line chemotherapy. Ofev is indicated in adults for the treatment of Idiopathic Pulmonary Fibrosis (IPF), for the treatment of other chronic fibrosing interstitial lung diseases (ILDs) with a progressive phenotype, or for the treatment of systemic sclerosis associated interstitial lung disease (SSc-ILD).

Nintedanib was first described as base in WO 01/27081, and its monoethansulfonate (esylate) form was described in WO 2004/013099.

Oral formulations of nintedanib with an immediate release profile were discussed in WO 2009/147212 and WO 2009/147220. WO 2009/147220 discloses on page 10 that presence of lecithin in the formulation is required to increase the dissolution rate as required. The formulations as described in WO 2009/147212 comprise a carrier, a thickener and optionally a glidant. The only glidant considered is lecithin. Also recent documents seem to require or at least trongly prefer lecithin to achieve acceptable pharmaceutical properties of nintedanib formulations (e.g., US 2017/0065529, WO 2020/079706).

The prior art thus consensually teaches that immediate release lipophilic formulations of nintedanib or a salt thereof require the presence of lecithin. The commercially available nintedanib formulations (Ofev and Vargatef) also use lecithin as the only glidant. Lecithin is usually extracted from natural sources, including soy or rapeseed, in such a way that it contains negligible amounts of protein. However, these negligible amounts of protein may still cause an allergic reaction in highly intolerant subjects.

### Disclosure of the Invention

In the present invention, it was surprisingly found that lipophilic formulation of nintedanib or a pharmaceutically acceptable salt thereof, with immediate release profile, can be achieved without the need to use lecithin.

The present invention thus provides a formulation of nintedanib or a pharmaceutically acceptable salt thereof, which comprises nintedanib or a pharmaceutically acceptable salt thereof, a non-ionic surfactant (glidant, emulsifier), and at least one further pharmaceutically acceptable excipient.

The further pharmaceutically acceptable excipient may include, for example, carriers, thickeners, solvents, fillers, binders.

Preferably, the present invention provides a formulation which comprises nintedanib or a pharmaceutically acceptable salt thereof, a carrier, a thickener, and a surfactant (glidant, emulsifier), wherein the surfactant is a non-ionic surfactant.

The formulation of the present invention is preferably free of lecithin.

The pharmaceutically acceptable salt of nintedanib is a salt of nintedanib with a pharmaceutically acceptable anion. Such anions may be anions of inorganic acids or anions of organic acids. Examples of suitable anions include acetate, aspartate, benzene sulfonate, benzoate, besylate, bicarbonate, bitartrate, bromide, camsylate, carbonate, chloride, citrate, decanoate, edetate, esylate, fumarate, gluceptate, gluconate, glutamate, glycolate, hexanoate, hydroxynaphthoate, iodide, isdthionate, lactate, lactobionate, malate, maleate, mandelate, mesylate, methylsulfate, mucate, napsylate, nitrate, octanoate, oleate, pamoate, pantothenate, phosphate, polygalcturonate, propionate, salicylate, stearate, succinate, sulfate, tartrate, teoclate, tosylate.

The pharmaceutically acceptable salt of nintedanib is preferably nintedanib esylate.

The carrier is preferably a lipid or a lipophilic carrier, more preferably selected from glycerol, acetylated monoglycerides, corn oil glycerides, caprylic-capric triglycerides, medium chain triglycerides, medium chain partial glycerides, caprylic/capric/linoleic triglycerides, cyprylic/capric/succinic triglycerides.

Especially preferably, the carrier is medium chain triglycerides.

Medium chain triglycerides are triglycerides with two or three fatty acids having an aliphatic chain of 6-12 carbon atoms. Typical fatty acids having an aliphatic chain of 6-12 carbon atoms are caproic acid, caprylic acid, capric acid, lauric acid. Medium chain triglycerides are typically produced from palm kernel oil or coconut oil, by fractionation or by interesterification.

Medium chain triglycerides are commercially available as, e.g., Miglyol^{®} 810, Miglyol^{®} 812, Miglyol^{®} 818, Miglyol^{®} 829, Miglyol^{®} 840, Labrafac^{™} lipophile WL 1349, Neobee^{®} M-5, Captex^{®} 300, Captex^{®} 355.

The thickener may preferably be selected from bees wax, glycerol monostearate, hydrogenated vegetable oil, partially hydrogenated vegetable oil, hard fat.

Especially preferably, the thickener is hard fat (adeps solidus). Hard fat is a mixture of glycerides of saturated fatty acids.

Hard fat is commercially available as, e.g., Gelucire^{®} 33/01, Gelucire^{®} 43/01, Softisan^{®} 378, Witepsol^{®} W35.

The non-ionic surfactants may be selected from polyglyceryl-3-dioleate, sorbitan monolaurate, propylene glycol monolaurate, propylene glycol monocaprylate, linoleoyl polyoxyl-6-glycerides, glyceryl caprylate/caprate.

Examples of suitable non-ionic surfactants include:

| Trade name | USPNF Name | Chemical Components |
|---|---|---|
| Plurol Oleique CC 497 | Polyglyceryl-3 dioleate | Polyglyceryl-3 esters of oleic acid (C18:1), the diester fraction being predominant. |
| MontaneTM20 | Sorbitan monolaurate | Sorbitan monododecanoate |
| Lauroglycol^{™} FCC | Propylene glycol monolaurate (type I) | Propylene glycol mono- and diesters of lauric (C12) acid. |
| Capryol 90 | Propylene glycol monocaprylate (type II) | Propylene glycol esters of caprylic acid (C8), mainly composed of monoesters and a small fraction of diesters. |
| Labrafil^{®} M 2125 CS | Linoleoyl Polyoxyl-6 glycerides | Mono-, di- and triglycerides and PEG-6 (MW 300) mono- and diesters of linoleic (C18:2) acid |
| Capmul^{®} MCM | Caprylic/capric mono- & diglycerides | Glyceryl Caprylate/Caprate |

Particularly preferred non-ionic surfactants are polyglyceryl-3-dioleate and/or glyceryl caprylate/caprate.

In some embodiments, the formulation of the present invention comprises 30 to 60 wt. % of nintedanib or a pharmaceutically acceptable salt thereof, 0.2 to 5 wt. % of the non-ionic surfactant, the balance being further pharmaceutically acceptable excipient(s).

In some embodiments, the formulation of the present invention comprises 30 to 60 wt. % of nintedanib or a pharmaceutically acceptable salt thereof, 30 to 60 wt. % of the carrier, 5 to 30 wt. % of the thickener, and 0.2 to 5 wt. % of the non-ionic surfactant.

Preferably, the amount of nintedanib or a pharmaceutically acceptable salt thereof (preferably nintedanib esylate) is 40 to 50 wt. %, more preferably 40 to 45 wt. %, most preferably about 43 wt. %.

Preferably, the amount of the carrier is 35 to 55 wt. %. In some preferred embodiments, the amount of the carrier is 35 to 40 wt. %.

In a preferred embodiment, the formulation comprises 35 to 55 wt. % of medium chain triglycerides, more preferably 35 to 40 wt. % of medium chain triglycerides, or 48 to 53 wt. % of medium chain triglycerides.

Preferably, the amount of the thickener is 5 to 20 wt. %. In some preferred embodiments, the amount of the thickener is 5 to 10 wt. %. In other preferred embodiments, the amount of the thickener is 14 to 20 wt. %.

In a preferred embodiment, the formulation contains 5 to 20 wt. % of hard fat, preferably 5 to 10 wt. % of hard fat or 14 to 20 wt. % of hard fat.

The amount of the non-ionic surfactant in the formulation is preferably from 0.3 to 2 wt. %, even more preferably 0.4 to 1.8 wt. %, and yet more preferably 1.3 to 1.7 wt. %.

In particularly preferred embodiments, the non-ionic surfactant is selected from Plurol Oleique and Capmul, and is present in the amount of 0.3 to 2 wt. %, even more preferably 0.4 to 1.8 wt. %.

In a particularly preferred embodiment, the formulation comprises 40-43 wt. % of nintedanib esylate, 36-40 wt. % of medium chain triglycerides, 17-19 wt. % of hard fat, and 0.9 to 1.6 wt. % of Plurol Oleique (polyglyceryl-3-dioleate).

The formulation of the present invention may be prepared by homogenizing the excipients, preferably by homogenizing the carrier, thickener and surfactant at a temperature of 20 to 60 °C (preferably at 30 to 50 °C, mixing them thoroughly, and transfering nintedanib or a pharmaceutically acceptable salt thereof into the mixture of excipients. The resulting mixture is then stirred thoroughly.

The formulation of the present invention is preferably filled into capsules, more preferably into soft capsules.

Therefore, the present invention provides a capsule comprising a capsule shell and a formulation as described above, said formulation being filled within the capsule shell.

The capsule shell may be selected from a hard gelatin shell, soft gelatin shell, hydroxypropylmethylcellulose shell, polyvinyl alcohor polymer shell, pullulan shell. Most preferred is soft gelatin shell.

An especially preferred soft gelatin shell comprises gelatin, glycerol and optionally pigments (for example, titanium dioxide, ferric oxide). Such soft gelatin shells are widely commercially available.

Another aspect is the formulation and/or capsule of the present invention for use in the treatment or prevention of cancer, immunologic diases or fibrotic disease.

In particular, the invention provides the formulation and/or capsule as described herein for use in the treatment or prevention of non-small cell lung cancer (NSCLC), Idiopathic Pulmonary Fibrosis (IPF), chronic fibrosing interstitial lung diseases (ILDs) or systemic sclerosis associated interstitial lung disease (SSc-ILD).

Yet another aspect is a method of treatment or prevention of a cancer, immunologic diases or fibrotic disease in a subject in need of such treatment, said method comprising the step of administering the formulation or the capsule of the invention to the said subject (in a therapeutically effective amount).

In this aspect, the invention provides a method of treatment or prevention of at least one of non-small cell lung cancer (NSCLC), Idiopathic Pulmonary Fibrosis (IPF), chronic fibrosing interstitial lung diseases (ILDs) or systemic sclerosis associated interstitial lung disease (SSc-ILD) in a subject in need of such treatment, said method comprising the step of administering the formulation or the capsule of the invention to the said subject (in a therapeutically effective amount).

The subject is preferably a mammal, more preferably a human, even more preferably an adult human.

The invention is further illustrated by way of examples. The examples should not be construed as limiting the scope of the invention in any way. The scope of the invention is determined by the wording of the claims.

### Brief description of drawings

Figure 1 is a dissolution graph of RLD formulation and formulations of Example 4.
Figure 2 is a dissolution graph of RLD formulation and formulations of Example 5.

### Examples

General procedure for preparing the formulations discussed in the examples:
Medium chain triglycerides, hard fat and surfactant were transferred to a homogenizer which was preheated to about 40 °C. The mixture was stirred at 2,500 rpm ± 500 rpm for about 20-30 minutes. Unless indicated otherwise, Softisan^{®} 378 was used as hard fat, and Miglyol^{®} 812N was used as medium chain triglycerides.
Nintedanib esylate was slowly added to the excipient mixture in the homogenizer and the resulting mixture was stirred at 4,700 rpm ± 700 rpm. After complete addition of nintedanib esylate, stirring was continued for another at least 30 minutes at 40 °C.

General procedure for preparing the soft gelating capsules:
Soft gelatin capsules, containing gelatin, glycerol, titanium dioxide, red ferric oxide and yellow ferric oxide, were filled with the formulation of nintedanib esylate prepared as described above.

### Example 1:

The table shows a composition of RLD (reference listed drug, comparative formulation) and a formulation according to the invention (formulation LP655-002). The amounts are in wt. %.

| Material | RLD | LP655-002 |
|---|---|---|
| Nintedanib Esylate | 43.00% | 43.00% |
| Medium chain triglycerides | 38.36% | 38.36% |
| Hard fat | 18.21% | 18.21% |
| Lecithin | 0.43% | - |
| Plurol Oleique CC 497 | | 0.43% |
| Total Percentage (%) | 100.00% | 100.00% |

### Example 2:

The table shows a composition of a formulation according to the invention. The amounts are in wt. %.

| Material | Composition -I |
|---|---|
| Nintedanib Esylate | 43.00% |
| Medium chain triglycerides | 47.03% |
| Hard fat | 9.11% |
| Plurol Oleique CC 497 or MontaneTM20 or Lauroglycol^{™} FCC or Capryol 90 or Labrafil^{®} M 2125 CS or Capmul^{®} MCM | 0.86% |
| Total Percentage (%) | 100.00% |

### Example 3:

The table shows a composition of a formulation according to the invention. The amounts are in wt. %.

| Material | Composition -II |
|---|---|
| Nintedanib Esylate, | 43.00% |
| Medium chain triglycerides | 41.00% |
| Hard fat | 15.00% |
| Capmul^{®} MCM or Plurol Oleique CC 497 | 1.00% |
| Total Percentage (%) | 100.00% |

### Example 4:

The table shows compositions of several formulations according to the invention. The amounts are in wt. %. In this example, various concentrations of Plurol Oleique and Capmul as surfactants (emulsifiers) were evaluated.

| Material | | LP655-009 | LP655-014 | LP655-016 | LP655-017 |
|---|---|---|---|---|---|
| Nintedanib Esylate | API | 43.00% | 43.00% | 43.00% | 43.00% |
| Medium chain triglycerides | Carrier | 47.03% | 37.93% | 41.00% | 50.00% |
| Hard fat | Thickener | 9.11% | 18.21% | 15.00% | 6.00% |
| Capmul^{®} MCM | Emulsifier | | | 1.00% | |
| Plurol Oleique CC 497 | Emulsifier | 0.86% | 0.86% | | 1.00% |
| Total Percentage (%) | | 100.00% | 100.00% | 100.00% | 100.00% |

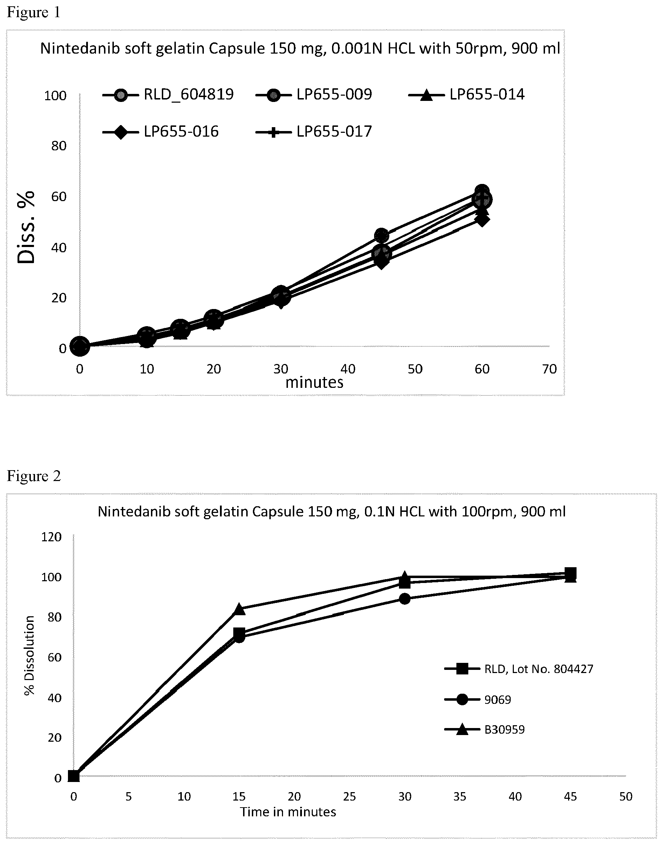

The following table shows dissolution profiles of the RLD and the batches prepared in this example. The dissolution profiles of samples are also shown in Figure 1.

The dissolution tests were performed on the formulation prepared in samples with sinker using 900 mL of 0.001 N HCl (pH 3.0) as a dissolution medium at the stirring speed of 50 rpm and at a temperature of 37 °C. A sample of the tested solution was taken at each time point and the dissolution rate was analyzed by HPLC.

| Time Points (min) | RLD Ofev soft capsules 150mg | LP655-016 | LP655-009 | LP655-017 | LP655-014 |
|---|---|---|---|---|---|
| | Dissolved (%) | Dissolved (%) | Dissolved (%) | Dissolved (%) | Dissolved (%) |
| 0 | 0 | 0 | 0 | 0.0 | 0 |
| 10 | 2 | 3 | 4 | 5 | 2 |
| 15 | 6 | 6 | 7 | 8 | 5 |
| 20 | 10 | 9 | 11 | 12 | 9 |
| 30 | 21 | 18 | 20 | 22 | 19 |
| 45 | 44 | 33 | 37 | 40 | 36 |
| 60 | 61 | 50 | 58 | 59 | 54 |

### Example 5:

The table shows compositions of RLD (comparative formulation) and two formulations according to the invention. The amounts are in miligrams and wt. %. The formulations were filled into soft gelatin capsules.

| Excipient | Batch 9069 | | Batch B30959 | | RLD | |
|---|---|---|---|---|---|---|
| | Quantity/ cap | %(w/w) | Quantity/ cap | %(w/w) | Quantity/ cap | %(w/w) |
| Nintedanib Esylate | 180.6 | 43.00% | 180.60 | 43.00% | 180.6 | 43.00% |
| Medium chain Triglycerides | 158.718 | 37.79% | 156.62 | 37.29% | 161.1 | 38.36% |
| Hard Fat | 76.482 | 18.21 % | 76.48 | 18.21% | 76.5 | 18.21% |
| Plurol Oleique CC497 | 4.2 | 1.00% | 6.30 | 1.50% | - | - |
| Lecithin liquid | - | - | - | - | 1.8 | 0.43% |
| Total | 420 | 100.00% | 420 | 100.00% | 420 | 100.00% |

Dissolution profiles of the capsules with formulations of this example are shown in Figure 2.
The dissolution tests were performed on the formulation prepared in samples with sinker using 900 mL of 0.1 M HCl (pH 1.0) as a dissolution medium at the stirring speed of 100 rpm and at a temperature of 37 °C according to the FDA-Recommended Dissolution Methods Web site. A sample of the tested solution was taken at each time point and the dissolution rate was analyzed by HPLC.

### Example 6: Comparative bioavailability analysis results for Nintedanib with Plurol under fasting condition:

Tested was formulation 9069, in comparison with the reference product Ofev^{®} 150 mg soft capsules - Germany.

| **Dependent** | **Ratio** |
|---|---|
| **Cmax (ng/mL)** | 96.56 |
| **AUCₜ (ng.h/mL)** | 97.07 |

| | |
|---|---|
| **Study Title** | Bioequivalence study of Nintedanib Soft Gelatin Capsules 150 mg in healthy, adult, human male subjects under fasting conditions. |
| **Study Design** | Open label, randomized, balance, three-treatment, three-period, three sequence, single-dose, crossover, pilot oral bioequivalence study with a wash period of at least 07 days in healthy, adult, human male subjects under fasting conditions. |
| **No. of Subjects** | 24 Subjects |
| **Housing** | Subjects were housed at least 10 hours prior to dosing the Clinical Pharmacology Unit until 48.00 hours post-dose study period. |
| **Wash Out Period** | At least 07 days |
| **Study Meals** | Subjects were fasted for at least 10.00 hours prior to dosing and 4.00 hours post-dose. Meals were served to subjects at 4.00, 8.00, 12.00, 24.00, 28.00, 32.00 and 36.00 hours post-dose (+30 minutes of scheduled time), in each study period. The meal plan was identical and standardized in all the study periods. |
| **Blood Sampling** | 28 sampling time points: Pre-dose, 0.250, 0.500, 1.000, 1.500, 2.000, 2.333, 2.667, 3.000, 3.333, 3.667, 4.000, 4.500, 5.000, 5.500, 6.000, 6.500, 7.000, 7.500, 8.000, 8.500, 9.000, 10.000, 12.000, 24.000, 36.000, 48.000 and 72.000 hours. |

### Example 7: Comparative bioavailability analysis results for Nintedanib with Plurol under fed condition

Tested was formulation 9069, in comparison with the reference product Ofev^{®} 150 mg soft capsules - Germany.

| **Dependent** | **Ratio** |
|---|---|
| **Cmax (ng/mL)** | 103.13 |
| **AUCₜ (ng.h/mL)** | 107.35 |

| | |
|---|---|
| **Study Title** | Bioequivalence study of Nintedanib Soft Gelatin Capsules 150 mg in healthy, adult, human male subjects under fed conditions. |
| **Study Design** | Open label, randomized, balance, three-treatment, three-period, three sequence, single-dose, crossover, pilot oral bioequivalence study with a wash period of at least 07 days in healthy, adult, human male subjects under fed conditions. |
| **No. of Subjects** | 18 Subjects |
| **Housing** | The subjects were housed from at least 11 hours prior to drug administration until after the 48 hours post dose in each period. |
| **Study Meals** | Following an overnight fast of at least 10 hours subjects were provided a high calorie and high fat breakfast at exactly 30 minutes prior to drug administration. |
| **Wash Out Period** | At least 07 days between treatments |
| **Blood Sampling** | 28 sampling time points: Pre-dose, 0.250, 0.500, 1.000, 1.500, 2.000, 2.333, 2.667, 3.000, 3.333, 3.667, 4.000, 4.500, 5.000, 5.500, 6.000, 6.500, 7.000, 7.500, 8.000, 8.500, 9.000, 10.000, 12.000, 24.000, 36.000, 48.000 and 72.000 hours. |

## Claims

1. A formulation of nintedanib or a pharmaceutically acceptable salt thereof, wherein the said formulation comprises nintedanib or a pharmaceutically acceptable salt thereof, a non-ionic surfactant, and at least one further pharmaceutically acceptable excipient.

2. The formulation according to claim 1, which is free of lecithine.

3. The formulation according to any one of the preceding claims, wherein the non-ionic surfactant is selected from polyglyceryl-3-dioleate, sorbitan monolaurate, propylene glycol monolaurate, propylene glycol monocaprylate, linoleoyl polyoxyl-6-glycerides and glyceryl caprylate/caprate.

4. The formulation according to any one of the preceding claims, wherein the non-ionic surfactant is polyglyceryl-3-dioleate and/or glyceryl caprylate/caprate.

5. The formulation according to any one of the preceding claims, which comprises 30 to 60 wt. % of nintedanib or a pharmaceutically acceptable salt thereof and 0.2 to 5 wt. % of the non-ionic surfactant.

6. The formulation according to any one of the preceding claims, wherein the further pharmaceutically acceptable excipients are a carrier and a thickener.

7. The formulation according to claim 6, wherein the carrier is selected from glycerol, acetylated monoglycerides, corn oil glycerides, caprylic-capric triglycerides, medium chain triglycerides, medium chain partial glycerides, caprylic/capric/linoleic triglycerides, cyprylic/capric/succinic triglycerides.

8. The formulation according to claim 6, wherein the carrier is medium chain triglycerides.

9. The formulation according to any one of claims 6 to 8, wherein the thickener is selected from bees wax, glycerol monostearate, hydrogenated vegetable oil, partially hydrogenated vegetable oil, hard fat.

10. The formulation according to any one of claims 6 to 8, wherein the thickener is hard fat.

11. The formulation according to any one of claims 6 to 10, wherein the formulation comprises 30 to 60 wt. % of nintedanib or a pharmaceutically acceptable salt thereof, 30 to 60 wt. % of the carrier, 5 to 30 wt. % of the thickener, and 0.2 to 5 wt. % of the non-ionic surfactant.

12. The formulation according to any one of the preceding claims, wherein the pharmaceutically acceptable salt of nintedanib is nintedanib esylate.

13. The formulation according to claim 6, wherein the formulation comprises 40-43 wt. % of nintedanib esylate, 36-40 wt. % of medium chain triglycerides, 17-19 wt. % of hard fat, and 0.9 to 1.6 wt. % of Plurol Oleique (polyglyceryl-3-dioleate).

14. A capsule comprising a capsule shell and a formulation according to any one of the preceding claims, said formulation being filled within the capsule shell.

15. The capsule according to claim 14, wherein the capsule shell is a soft gelatin shell.

16. The formulation according to any one of claims 1-13 and/or the capsule according to claims 14 or 15 for use in the treatment or prevention of cancer, immunologic diseases or fibrotic disease.

17. The formulation according to any one of claims 1-13 and/or the capsule according to claims 14 or 15 for use in the treatment or prevention of non-small cell lung cancer, idiopathic pulmonary fibrosis, chronic fibrosing interstitial lung diseases or systemic sclerosis associated interstitial lung disease.
